# EUROPÄISCHE PATENTSCHRIFT

(11) **EP 1 514 574 B1**
(45) Veröffentlichungstag und Bekanntmachung des Hinweises auf die Patenterteilung: **27.07.2011**
(21) Anmeldenummer: 04105455.2
(22) Anmeldetag: 11.09.2001
(51) Int. Cl.: A61M 37/00

(54) **Narbenfreie Entfernung von Tätowierungen**
Scar-free tattoo removal
Détatouage sans cicatrice.

(30) Priorität: 04.11.2000 DE 10054718; 04.11.2000 DE 20018848 U; 13.11.2000 DE 10056114
(43) Veröffentlichungstag der Anmeldung: 16.03.2005
(62) Teilanmeldung aus: 01965277.5
(73) Patentinhaber: Simpson-Birks, Barbara, 34800 Clermont L'Herault (FR); Simpson-Birks, Richard, 34800 Clermont L'Herault (FR)
(72) Erfinder: Malodobry, Wolfgang, 53870 Euskirchen (DE)
(74) Vertreter: von Kreisler Selting Werner

(56) Entgegenhaltungen:
- WO-A-89/12440
- DE-C- 385 666
- FR-A- 609 177
- NL-A- 8 901 972

## Beschreibung

Gegenstand der Erfindung ist das narbenfreie Entfernen, (nicht gemeint sind die Melaninnarben), von nicht natürlichen tätowierten Farbpigmenten in der menschlichen oder tierischen Haut.

Beim modernen Tätowieren wird die Farbe, gemeint sind in Zukunft die Tattoofarben, mit der Maschine in die Haut gestochen. Durch die gleichlaufende Pistole wird bei professionellen Tätowierungen die Farbe in die Haut gestochen. Die Haut baut sich aus 3 verschiedenen Schichten auf:
1. Oberhaut
2. Lederhaut
3. Unterhaut

Die Oberhaut (Epidermis) ist etwa 0,5 mm dick und unterteilt sich weiter in verschiedene Schichten.

Die äußere hiervon ist die Hornschicht (Hornhaut) die etwa 10 bis 20 Zeltschichten (0,015 mm) dick ist. Deren toten und verhornten Zellen werden ständig nach außen abgestoßen und müssen deshalb durch Zellproduktion in der Keimschicht ersetzt werden. Dieser Übergang erfolgt durch Zusammenrücken und Abplatten der Zellen, das Auflösen ihrer Kerne sowie und Einlagerung von Keratinkörnchen in ihr Protoplasma. Die so entstehende Körnerschicht geht an dicken Hautstellen durch Vernetzung der Keratinkörnchen und Ausbildung von faserigen Strukturen zu einer stark lichtbrechenden Masse in die Glanzschicht und zuletzt in die Hornhaut über.

Die Lederhaut wird vom mittleren Keimblatt gebildet. Sie besteht aus Bindegewebe und enthält Gefäße und Nerven. An einigen Stellen sogar Muskulatur. Die Füllung der in der Lederhaut enthaltenen Kapillargefäße bedingt die Rosatönung der Haut. In der Netzschicht der Lederhaut liegen die Schweißdrüsen sowie die größeren Gefäße und Nerven. Beim Tätowieren wird die Farbe etwa 1 bis 1,5 mm tief in die Lederhaut gestochen.

Unter der Lederhaut liegt die Unterhaut. In sie eingebettet dient das Unterhautfettgewebe der Wärmeisolation des Körpers, als Druckpolster sowie der Speicherung von Reservestoffen.

Die Farbe wird aus der Handelsflasche in Einwegtöpfchen geschüttet. Aus diesen Töpfchen heraus nimmt der Tätowierer während des Stechens mit der Pistole die Farbe. Die Pistole selbst wird bei jedem Kunden mit frisch desinfizierten Nadeln und Griffstücken versehen. Die zu tätowierende Stelle wird mit einem Einwegrasierer rasiert, mit Desinfektionsmittel gereinigt und eingefeuchtet. Rasiert wird die Stelle damit durch die Nadeln keine Haare mit unter die Haut gestoßen werden. Hierdurch würden sich evtl. Infektionen oder Entzündungen ergeben. Bei einem Farbwechsel und nach dem Gebrauch der Pistole werden Nadel und oft auch das Griffstück gereinigt.

Auf der Maschine befindet sich kein Tank, sondern die Nadel wird in regelmäßigen Abständen immer wieder in das (Einweg-)Töpfchen mit Farbe getaucht. Wird die Farbe zu tief gestochen, verschwimmt die Farbe unter der Haut, (sog. Blow Out). Wenn die Farbe nicht tief genug eingedrungen ist, kann es sein, dass die Farbe mit der Zeit verblasst. Für die Maschinen gibt es verschiedene Aufsätze für verschiedene Zwecke. Feine Konturen werden in der Regel mit einem Aufsatz von mehreren, insbesondere 3 oder 5 Nadeln mit 0,25 bis 0,40 mm Durchmesser gestochen, Flächen dagegen mit bis zu 10 bis 14 Nadeln gleichzeitig. Diese Nadeln werden in Handarbeit aus medizinisch geeignetem Stahl (Nirosta® oder V2A) angefertigt und vor dem Einsatz unter dem Mikroskop auf Beschädigungen an der Spitze untersucht. Die Nadeln werden dann nach Bedarf entweder von dem Tätowierer selbst oder einem Lieferanten gebündelt und mit einem speziellen Lot auf einen Träger gelötet. So kann man die Nadelkombination nach Wunsch des Tätowierers wählen.

Praktisch werden nur elektrische Maschinen eingesetzt. Dieses Verfahren ist im Gegensatz zu den älteren relativ schmerzarm und eignet sich aufgrund der gleichmäßigen Vibrationen gut für feine Bilder. Die Tattoopistole kann von dem Tätowierer nahezu wie ein Malpinsel geführt werden. Das Gerät funktioniert analog einer elektrischen Türklingel mit angeschlossener Nadel. Am Kopf der Maschine befinden sich ein oder zwei Magnetspulen, die durch Zuführung von Strom aktiviert werden. Die über den Spulen befindliche Metallfeder wird von den Spulen angezogen, biegt sich nach unten und bewegt dadurch die angeschlossene Nadel. Wenn die Metallfeder nach unten gezogen wird, unterbricht sie den Stromkreis und die Magnetspulen werden wieder ausgeschaltet; die Feder schwingt wieder nach oben. Dort schließt sie wieder den Stromkreis und das gleiche beginnt wieder von vorne: Dadurch Wird die an der Feder angeschlossene Nadeln auf und ab bewegt. Manche Tätowierer erhöhen die Spannung der Feder, indem sie zusätzlich Gummibänder um die Pistole spannen.

Die dadurch konstant gehaltene Stichfrequenz gewährt einen gleichmäßigen Druck und gleichbleibenden Farbstich. Die Geschwindigkeit, die an dem Netzteil der Maschine eingestellt wird, liegt in der Regel zwischen 30 und 40 Stichen pro Sekunde.

Für die Entfernung von Tattoos gibt es unterschiedliche Verfahren:

Eine relativ verbreitete Methode ein Tattoo verschwinden zu lassen ist das "Cover-Up". Hierbei wird das alte Bild in ein neues Bild eingebunden und übertätowiert.

Der bekannte Beginn der Tattoo-Entfernung liegt irgendwo in der Zeit um 54 n.Chr. Ein griechischer Arzt hatte eine chemische Methode entwickelt, die zum Absterben des gestochenen Hautgewebes führte. Hierzu diente eine Mischung aus Knoblauchzwiebeln und Kantharidin. Hierbei handelt es sich um ein Fliegendrüsensekret. Diese Paste wurde auf die Haut aufgetragen um den gewünschten Effekt zu erreichen.

Nicht zu empfehlen ist das einfache Ausschneiden der Tätowierung. Hierbei wird die betreffende Stelle ausoperiert oder ausgestanzt und vernäht. Dieses Verfahren ist nur bei kleineren Tattoos anwendbar, führt aber auch bei diesen kleinen Eingriffen in aller Regel zu unschönen Narben.

Eine sehr rabiate Methode dagegen ist das mechanische Verfahren, die sogenannte Dermabrasion, bei dem die Haut schlicht abgehobelt bzw. abgeschliffen wird. Hierbei entstehen naturgemäß Narben. Oft ist es so, dass Pigmente, die in unterschiedlichen Hautschichten liegen, nicht mitentfernt werden. Es bleibt zur Narbe ein dunkler Schatten der durchscheinenden Überreste der Tätowierung.

Ein weiteres Verfahren besteht in der tangentialen Excision und Deckung mit "Spalthaut-Transplantat" Die betreffende Hautschicht wird unter Vollnarkose herausgeschnitten, wobei versucht wird, möglichst viel der darunter liegenden Hautschicht zu erhalten. Die offene Stelle wird mit Spalthaut abgedeckt und über Monate hinweg durch Kompressionsverbände vor unnötiger Narbenbildung bewahrt und an die Umgebung angepasst.

Die Entfernung einer Tätowierung mittels Laser, wie beispielsweise in US-A-5,984,915 oder US-A-5,000,752 beschrieben hat sich mittlerweile durchgesetzt.

Diese saubere und im Handling relativ einfache Behandlung ist aber auch nicht perfekt. Die Behandlungen sind in der Regel kostenaufwendig und dauern ziemlich lange bis ein annehmbares Ergebnis erzielt wurde.

Bei der Laserbehandlung macht man sich das Prinzip der Lichtreflektion der Farbteilchen zunutze. Bei den Tätowierungen handelt es sich bekanntermaßen um anorganische oder organische Farbpigmente, die in Form von Agglomeraten in der Haut eingelagert sind. Diese Pigmente werden insbesondere in der Lederhaut dauerhaft eingelagert, da sie von dem Immunsystem nicht ab- und ausgesondert werden.

Die sichtbare Tätowierung stellt nun die Lichtreflektion der Pigmentteilchen in der ihnen eigenen Wellenlänge des Lichtes dar. Werden diese Agglomerate nun mit dem energiereichen Laser in ihrer ureigenen "Wellenlänge" bestrahlt, absorbieren sie die Strahlung, werden zerstört und die Reste von dem Abwehrsystem als Fremdkörper abtransportiert. Das umliegende Hautgewebe soll auf diese Wellenlänge des Lasers nicht reagieren und unverletzt bleiben.

Nach der Behandlung ist die entsprechende Stelle etwas heller als die Umgebung, aber durch die natürliche Pigmentierung der Haut wird dies wieder reguliert und gleicht sich nach einiger Zeit aus.

Die Anzahl und die Dauer der Laser-Behandlungen hängt nun von einer Reihe von Faktoren ab:
- Größe der Tätowierung
- Tiefe
- Farbe
- Zustand und Stärke des Immunsystems
- Regelmäßigkeit der Behandlungen
- Nachbehandlungen
- Qualität der Tätowierung

Die Qualität ist von entscheidender Rolle, weil bei Handstichen die Farbpigmente in verschieden tiefen Hautschichten stecken und unregelmäßig verteilt sind, wohingegen maschinengestochene Bilder regelmäßig in einer Hautschicht sein sollten.

Bei Laientätowierungen (einfarbig blau-schwarz) wird bei einer Größe von 10 x 10 cm von 4 - 8 Sitzungen, bei einer Profitätowierung (farbig) von 8 - 12 Sitzungen ausgegangen. Jede Sitzung ist kostenintensiv.

Eine der neuesten Behandlungen ist die sogenannten Diathermie. Hierbei nutzt man nicht die Wellenlängeneigenschaft der Farbe, sondern setzt auf thermische Behandlung der Haut. Durch hochfrequente elektrische Ströme wird elektrische Energie in der Haut in Wärmeenergie umgewandelt. Dieses führt je nach Stärke zu einem Verkochen oder Verdampfen der Zellflüssigkeit. Die Zellen sterben dadurch ab. Die, Technik ist hier ähnlich wie beim Stechen selber. Hierbei werden ja durch das Einstechen der Nadeln die obersten Hautzellen zerstört und die Farbpigmente in die intakten Zellen eingelagert. Bei der Behandlung mit dem entsprechenden Diathermie-Gerät werden nun diese noch intakten Zellen nicht mechanisch, sondern durch Hitze zerstört. Die flüssige Zellsubstanz verdampft und die eingelagerten Farbpigmente werden mit den abgestorbenen Zellen abgestoßen. Eine Gefahr für die restliche Haut besteht hier aufgrund der geringen Stromstärke nicht. Narben sollen keine zurückbleiben. Durch das Abtöten und das Verletzen der Haut durch Hitzeeinwirkung wird der Prozess der Zellteilung angeregt. Die Haut versucht sich aufgrund der Verletzung zu heilen. Die abgestorbene farbige Haut wird nur nach außen geschoben um darunter gesunde Haut nachwachsen zu lassen. Nach einiger Zeit bildet sich auf der Haut diese typische Schorfschicht, die nun die Farbpigmente enthält. Der Vorteil dieses Verfahrens gegenüber dem Laser soll darin liegen, dass hier alle Farbbereiche gleichermaßen erfasst werden und nicht durch Wellenlängenmodulation die einzelnen Farbbereiche separat angegriffen werden müssen. Die Farbpigmente werden dabei nicht zerstört. Der Zeitraum und die Kosten hängen stark vom Aufwand und Dauer der Behandlung ab. Es wird quasi Schicht für Schicht der Haut behandelt, was besonders bei alten und farbintensiven Tattoos langwierig sein kann. Von 5 bis 8 Behandlungen ist auch bei alten und tiefen Tätowierungen die Rede. Die DE 43 08 824 C1 betrifft ein entsprechendes Verfahren, betont jedoch, dass sich naturgemäß eine Narbe bildet, nachdem die Schorfschicht abgefallen ist. Es wird vorgeschlagen, die Narben durch Abschleifen der Haut zu entfernen.

Das Waterjet-Cutting ist ein Verfahren, das normalerweise für das Schneiden von Gewebe verwandt wird. Durch einen feinen Wasserstrahl, der in Durchmesser, Druck und Pulsfrequenz geändert sowie mit verschiedenen Zusätzen versehen werden kann, werden die Farbpigmente unter der Haut weggespült.

Die Haut wird an der entsprechenden Stelle angeschnitten und angehoben, um überhaupt an die Farbpigmente zu kommen. Es bleiben also Narben zurück. Auch hier spielt es, wie in der Diathermie, keine Rolle, um welche Farbpigmentierung es sich handelt. Die Farbpigmente verbleiben nicht im Körper, sondern werden herausgespült. Auch großflächige Tätowierungen sollen kein Problem sein. Diese Methode befindet sich hinsichtlich Tätowierungen noch in der Probephase. Über Behandlungsdauer und -kosten ist nichts bekannt. Angesichts der Tatsache aber, dass ein stationärer Aufenthalt erforderlich ist, werden sich die Kosten wahrscheinlich sehr hoch sein.

US-A-5,833,649 beschreibt ein Verfahren zur Maskierung von Tätowierungen, bei dem eine bestehende Tätowierung nur abgedeckt, nicht jedoch entfernt wird.

WO 89/12440 offenbart ein Verfahren zur Entfernung von Tätowierungen mittels einer Flüssigkeit aus Kokosnussmilch, Milchsäure und Zwiebel saft.

Die Aufgabe der vorliegenden Erfindung besteht somit in der Bereitstellung eines kostengünstigen Verfahrens zur narbenfreien Entfernung von Tätowierungen der menschlichen oder tierischen Haut.

Die vorgenannte Aufgabe wird in einer ersten Ausführungsform erfindungsgemäß gelöst durch ein Verfahren gemäß Anspruch 1. Gemäß der vorliegenden Erfindung wird eine Tätowierung dadurch entfernt, dass man die relativ großen Farbpigment-Agglomerate direkt in der Lederhaut an Ort und Stelle mechanisch zerstört. Die bei der mechanischen Zerstörung entstehenden kleineren Bruchstücke der Agglomerate können dann beim natürlichen Heilungsprozess der Haut mit abgetragen werden.

Wenn im Sinne der vorliegenden Erfindung das Werkzeug im wesentlichen senkrecht zur Hautoberfläche durch diese hindurchdringen soll, so ist hierin lediglich eine qualitative Differenzierung von mechanischen Hautabschälungsverfahren zu sehen, bei denen im wesentlichen waagerecht unterhalb der Hautoberfläche die darüber liegenden Bereiche praktisch abgeschnitten werden und nach Entfernung der Tätowierung gegebenenfalls wieder aufgelegt werden. Der Begriff "senkrecht zur Hautoberfläche" kennzeichnet somit im wesentlichen einen Winkel von 0 bis 90°, insbesondere bis 60°, bezogen auf ein Lot auf der Hautoberfläche.

Die in die Farbpigment-Agglomerate eingebrachten Nadeln werden nach der mechanischen Einwirkung aus dem Farbpigment-Agglomerat wieder entfernt, können jedoch gegebenenfalls auch erneut beliebig oft wieder in das Agglomerat eingeführt werden. Hierdurch ist eine Perforierung des Farbstoffagglomerats möglich. Die Größe der Nadeln sollte möglichst in der gleichen oder kleineren Größenordnung der Farbpigment-Agglomerate liegen. Nadeln im Sinne der vorliegenden Erfindung umfassen beispielsweise Bohrer oder Hohlnadeln zur Injektion oder zum Absaugen von Gasen oder Flüssigkeiten.

Narbenfrei im Sinne der vorliegenden Erfindung bedeutet die Freiheit von Narben bei der Beobachtung mit dem bloßen Auge.

Um die mechanische Zerstörung der Farbpigment-Agglomerate zu verbessern, sollte die einzubringende Nadel eine relativ raue oder scharfkantige Oberfläche aufweisen. Während bei der Tätowierung selbst diese reit einer möglichst glatten Nadel mit glatter Oberfläche die Farbe in die Zellen der Lederhaut eingebracht wird, so ist es im Sinne der vorliegenden Erfindung bei der Entfernung der Tätowierung besonders bevorzugt, die Farbpigment-Agglomerate mit einer rauen Oberfläche in Kontakt zu bringen, damit hierdurch der natürliche Heilungsprozess beschleunigt wird.

Besonders bevorzugt im Sinne der vorliegenden Erfindung ist es, zum mechanischen Zerstören der Farbpigment-Agglomerate eine oder mehrere Nadeln einzusetzen. Diese Nadeln können in an sich bekannter Weise mit einer Tätowierpistole verbunden sein. Durch das Bearbeiten der Haut mit einer entsprechenden Tätowierpistole und den geeigneten Nadeln, beispielsweise mit einem Durchmesser von 0,001 bis 0,5 mm, insbesondere 0,01 bis 0,3 mm ist es somit möglich, Tätowierungen narbenfrei zu entfernen. Übliche Tätowiernadeln haben eine relativ glatte Oberfläche, so dass es im Sinne der vorliegenden Erfindung besonders bevorzugt ist, Tätowiernadeln entsprechend aufzurauen und einzusetzen. Hierbei ist es selbstverständlich möglich, bereits industriell vorgefertigte Nadeln einzusetzen.

Darüber hinaus ist es jedoch auch möglich, anstelle eines üblichen Farbtöpfchens mit einer Farbe zum Tätowieren, hier zur Tätowierungsentfernung ein entsprechendes Reib- oder Schleifmittel, wie Quarz oder Diamantstaub einzusetzen, um die mechanische Zerstörung der Farbpigment-Agglomerate zu erhöhen.

Ähnlich, wie bei der Diathermie werden die Farbpigment-Agglomerate nach außen geschoben, um darunter gesunde Haut nachwachsen zu lassen oder in die Unterhaut befördert, wo diese durch das Immunsystem entsorgt werden. Auch hier kann sich auf der Haut nach einiger Zeit eine typische Schorfschicht bilden, die Farbpigmente enthält. Bei der erfindungsgemäßen Behandlung wird jedoch keine flüssige Zellsubstanz verdampft. Die Vitalität der Zelle soll möglichst erhalten bleiben. Auch umliegende Bereiche außerhalb der Farbpigment-Agglomerate werden nicht thermisch beaufschlagt, so dass das volle Wundheilungsvermögen der Haut zur Verfügung steht. Dies steht im deutlichen Gegensatz zur Diathermie, die die Verbrennung oder Verbrühung von umliegenden Zellen nicht vermeiden kann. Die hierbei frei werdenden Stoffe bewirken eine hohe Durchlässigkeit der umliegenden Gewebe so dass Blutplasmaflüssigkeit ins Gewebe austritt. Das erfindungsgemäße Verfahren kommt jedoch ohne die thermische Einwirkung aus und schädigt daher die Haut praktisch nicht.

Die übliche Wundheilung fördert die Farbpigment-Agglomerat-Bruchstücke an die Hautoberfläche, wo diese dann abgeschilfert werden.

Im Sinne der vorliegenden Erfindung wird das Verfahren in der Weise angewendet, dass man vorher, während oder nach der mechanischen Zerstörung der Farbpigment-Agglomerate sogenannte Hautreizstoffe auf die Hautoberfläche oder direkt in die Agglomerate oder Bruchstücke der Farbpigment-Agglomerate einbringt. Herbert P. Fiedler definiert in "Lexikon "der Hilfsstoffe für Pharmazie, Kosmetik und angrenzende Gebiete", 4. Auflage, 1996 unter dem Stichwort "Hautirritationen", Hautreizstoffe als chemische Stoffe, die, wenn sie mit der Humanhaut in Berührung kommen, eine Hautreizwirkung ausüben. Hautreizstoffe im Sinne der vorliegenden Erfindung können somit einer primäre Reizwirkung aufweisen, die sofort nach dem Kontakt mit der Haut auftritt. Weniger geeignet im Sinne der vorliegenden Erfindung sind Hautreizstoffe, in denen sich Schädigungen erst nach länger dauernder Einwirkung des hautreizenden wirkenden Stoffes in unterschwelligen Dosen in Form eines degenerativen Ekzems bemerkbar machen. Besonders bevorzugt im Sinne der vorliegenden Erfindung werden somit Hautreizstoffe eingesetzt, die als Hautreizmittel dienen.

Fiedler loc. cit. definiert eine in den USA offiziell anerkannte Skala zur Prüfung der hautreizenden Wirkung. Die am stärksten hautreizenden Stoffe werden hier als korrosiv, hochgefährlich beschrieben, die mit einem Warnhinweis versehen sein müssen. Derartige Hautreizstoffe werden kaum für die vorliegende Erfindung eingesetzt werden, jedenfalls nicht in reiner Form. Gleiches gilt für primäre Hautreizstoffe, die ebenfalls hochgefährlich sind und Warnhinweise tragen müssen. Hautreizstoffe, die ein Potential für eine schwere Hautirritation haben, sollten ebenfalls im Sinne der vorliegenden Erfindung weniger geeignet sein. Hautreizstoffe, die kein hautreizendes Potential aufweisen, sind in Bezug auf die Wundheilung nicht von Bedeutung, es sei denn, dass die Anwesenheit als Füllstoff der Zelle die Wundheilung verzögert. Füllstoffe umfassen beispielsweise Feststoffe im Sinne der vorliegenden Erfindung, wie Kochsalz, Diamantstaub oder Quarzsand.

Die Hautreizstoffe können nicht nur in fester Form, sondern auch in flüssiger Form auf die Hautoberfläche oder direkt in die Farbpigment-Agglomerat-Bruchstücke eingebracht werden. **Im** Sinne der vorliegenden Erfindung **werden** Hautreizstoffe **eingesetzt**, die aus verdünnten wässrigen Lösungen, Dispersionen und Emulsionen der Hautreizstoffe bestehen. Die chemische Natur der Hautreizstoffe ist nicht auf eine bestimmte Klasse von Verbindungen beschränkt. So können beispielsweise verdünnte wässrige Lösungen von Milchsäure, Natriumhydrazin, Kochsalz, Aminosäuren, Fruchtsäuren eingesetzt werden, die gegebenenfalls geringe Mengen an Oxidationsmitteln enthalten. Durch einfache Reihenversuche lässt sich das hautreizende Potential ohne weiteres feststellen und somit eine entsprechende Auswahl treffen.

Neben der mechanischen Zerstörung ist es selbstverständlich auch möglich, die im Stand der Technik bekannten Verfahren zur Entfernung von Tätowierungen zeitgleich oder zeitversetzt mit einzusetzen, sofern dabei im Einzelfall nicht der Behandlungserfolg in Frage gestellt wird.

## Patentansprüche

1. Kosmetisches Verfahren zur narbenfreien Entfernung von Tätowierungen der menschlichen oder tierischen Haut durch Behandlung der Haut mit einem Werkzeug, das im wesentliche senkrecht zur Haut in die in der Haut vorhandenen Agglomerate von anorganischen und/oder organischen Farbpigmenten eingeführt wird, wodurch die Agglomerate mechanisch zerstört werden, wobei man vor, während oder nach der mechanischen Zerstörung der Agglomerate Hautreizstoffe auf die Hautoberfläche und/oder in die Agglomerate einbringt, die ausgewählt sind aus verdünnten wässrigen Lösungen, Dispersionen und Emulsionen.

2. Verfahren nach Anspruch 1, **dadurch gekennzeichnet, dass** man als Werkzeug Nadeln mit einer rauen Oberfläche einsetzt.

3. Verfahren nach Anspruch 2, **dadurch gekennzeichnet, dass** man Nadeln mit einem Durchmesser von 0,001 bis 0,5 mm, insbesondere 0,01 bis 0,3 mm einsetzt.

## Claims

1. A cosmetic method for the scarless removal of tattoos from the human or animal skin by treating the skin with a tool that is inserted in a direction essentially perpendicular to the skin into the agglomerates of inorganic and/or organic color pigments present in the skin, whereby the agglomerates are mechanically destroyed, wherein skin irritants selected from diluted aqueous solutions, dispersions and emulsions are applied to the skin surface and/or introduced into the agglomerates before, during or after the mechanical destruction of the agglomerates.

2. The method according to claim 1, **characterized in that** needles having a rough surface are employed as said tool.

3. The method according to claim 2, **characterized in that** needles having a diameter of from 0.001 to 0.5 mm, especially from 0.01 to 0.3 mm, are employed.

## Revendications

1. Procédé cosmétique pour l'élimination sans cicatrices des tatouages de la peau humaine ou animale par le traitement de la peau avec un outil qui est enfoncé pratiquement perpendiculairement à la surface de la peau dans les agglomérats de pigments colorés minéraux et/ou organiques qui se trouvent dans la peau, ainsi détruisant mécaniquement les agglomérats, dans lequel des produits irritant la peau choisis parmi les solutions aqueuses diluées, les dispersions et les émulsions sont appliqués sur la surface de la peau et/ou dans les agglomérats avant, pendant ou après la destruction mécanique des agglomérats.

2. Procédé selon la revendication 1, **caractérisé en ce que** des aiguilles ayant une surface rugueuse sont utilisées comme outil.

3. Procédé selon la revendication 2, **caractérisé en ce que** des aiguilles ayant un diamètre de 0,001 à 0,5 mm, en particulier de 0,01 à 0,3 mm, sont utilisées.
